# EUROPEAN PATENT APPLICATION

(11) **EP 3 144 717 A1**
(43) Date of publication of application: **22.03.2017**
(21) Application number: 16188303.8
(22) Date of filing: 12.09.2016
(51) Int. Cl.: G02B 27/01

(54) **WINDSCREEN DISPLAY SYSTEM**

(30) Priority: 15.09.2015 GB 201516289
(71) Applicant: Ford Global Technologies, LLC, Dearborn, MI 48126 (US)
(72) Inventor: QUARTA, Markus, London, E20 1DQ (GB); KELLY, Steven, London, EC2A 3HP (GB); HOGGARTH, Marcus, St. Albans, Hertfordshire AL1 4EP (GB); JARVIS, Alex, London, E3 2GY (GB)
(74) Representative: Dowling, Andrew

(57) **Abstract**

A windscreen display system for a motor vehicle, the windscreen display system comprising: a first projector configured to project a first image onto a driver's side of a windscreen so that light from the first projector is reflected by the windscreen towards the driver; a second projector configured to project a second image onto a passenger's side of the windscreen so that light from the second projector is reflected by the windscreen towards the passenger; and one or more privacy filters disposed between the windscreen and the first and second projectors, the privacy filters configured to limit the viewing angle of the light emitted from the first and second projectors such that, after passing through the privacy filters and reflecting from the windscreen, light rays from the first projector are substantially in line with the driver and light rays from the second projector are substantially in line with the passenger.

## Description

### Technical Field

This disclosure relates generally to a windscreen display system for a motor vehicle and particularly, but not exclusively, relates to a windscreen display system configured to display two images, one for a driver and one for a passenger, in which the driver's image is obscured from the passenger and vice versa.

### Background

The central console of a vehicle may be provided with a navigational display that may display route data and positional data provided by a satellite navigational system. However, the position of such navigational displays results in the driver's attention being diverted away from the windscreen and thus the road and traffic ahead. By contrast, portable navigational devices may be more conveniently mounted onto the windscreen or on top of the dashboard. However, such portable navigational devices may partially obscure the driver's view through the windscreen.

Accordingly, it is desirable for the driver of a motor vehicle to be able to view navigational information on the windscreen of the vehicle with the navigational data being overlaid onto the windscreen in such a way that it does not obscure the driver's view.

In addition, a passenger of the motor vehicle may wish to view media-based content. Previously-proposed media displays, such as portable DVD players and tablets, have small display screens and focussing on such screens can induce motion sickness. To address this and to provide a more immersive experience for the passenger, such media-based content may be viewed on the windscreen of the vehicle. However, it is desirable that such content should not distract the driver. Equally, the passenger may not want their media viewing to be disturbed by the driver's navigational data.

### Statements of Invention

According to an aspect of the present disclosure, there is provided a windscreen display system for a motor vehicle, the windscreen display system comprising:
a first projector configured to project a first image onto a driver's side of a windscreen so that light from the first projector is reflected by the windscreen towards the driver;
a second projector configured to project a second image onto a passenger's side of the windscreen so that light from the second projector is reflected by the windscreen towards the passenger; and
one or more privacy filters disposed between the windscreen and the first and second projectors, the privacy filters configured to reduce a viewing angle of the light emitted from the first and second projectors such that, after passing through the privacy filters and reflecting from the windscreen, the first image is not viewable by the passenger and the second image is not viewable by the driver.

This arrangement advantageously allows the driver to view the first image without being distracted by the second image for the passenger and vice versa. Furthermore, this arrangement can use existing windscreen technology and as such reduces the cost in the event of the windscreen needing replacing.

The privacy filters may substantially block light outside a particular viewing angle. Light rays from the first projector may disperse from the privacy filter within a viewing angle that includes the driver, but excludes the passenger. Light rays from the second projector may disperse from the privacy filter within a viewing angle that includes the passenger, but excludes the driver.

The windscreen display system may further comprise one or more mirrors configured to redirect light emitted from the first and/or second projectors in the direction of the windscreen. One or more of the privacy filters may be provided between the one or more mirrors and the windscreen. The privacy filters may form the last layer through which light from the projectors passes before reflecting on the windscreen. The privacy filters may be spaced apart from the projector. The privacy filters may be spaced apart from the windscreen.

One mirror common to each of the first and second projectors may be provided. For example, such a mirror may be provided across the width of the vehicle. Alternatively, one mirror may be provided for each of the first and second projectors. In either case further mirrors may be provided to further redirect the light from the projectors.

One or more of the mirrors may be curved. The curvature may correct any distortion resulting from a non-flat windscreen such that after reflection by the windscreen the first or second images appear to the respective driver or passenger to be undistorted.

The first and/or second projectors may be configured to project a distorted image that after reflection by the windscreen appears to the respective driver or passenger to be undistorted. In other words, the images projected by the projectors may be distorted in such a way so as to counteract the windscreen not being flat.

One or more of the privacy filters may be provided on a top of a dashboard of the vehicle. The privacy filters may be flush with the dashboard.

The windscreen display system may comprise a privacy filter common to each of the first and second projectors. For example, such a privacy filter may be provided across the width of the vehicle. Alternatively, the windscreen display system may comprise one privacy filter for each of the first and second projectors.

The windscreen display system may comprise a windscreen comprising a tinted layer on an inner surface of the windscreen. Such a tinted layer may reduce reflections from an outer surface of the windscreen. The tinted layer may comprise a coating or film applied to the windscreen.

The windscreen display system may comprise one or more rear projection display layers. The rear projection display layers may be disposed between the projectors and the privacy filters.

The windscreen display system may further comprise one or more transparent layers to which the rear projection display layers and privacy filters may be attached. The transparent layer may be provided on the dashboard top. The privacy filter may be provided on the top surface of the transparent layer. The rear projection display layer may be provided on a bottom surface of the transparent layer.

The first image may comprise navigational information. The second image may comprise media content. The first and/or second images may comprise moving and/or still images.

A vehicle may comprise the above-mentioned windscreen display system.

According to a further aspect of the present disclosure there is provided a method of displaying images on a windscreen of a motor vehicle, the method comprising:
projecting a first image onto a driver's side of a windscreen with a first projector so that light from the first projector is reflected by the windscreen towards the driver;
projecting a second image onto a passenger's side of the windscreen with a second projector so that light from the second projector is reflected by the windscreen towards the passenger; and
limiting the viewing angle of the light emitted from the first and second projectors with one or more privacy filters disposed between the windscreen and the first and second projectors, the privacy filters being configured to limit the viewing angle of the light emitted from the first and second projectors such that, after passing through the privacy filters and reflecting from the windscreen, the first image is not viewable by the passenger and the second image is not viewable by the driver.

To avoid unnecessary duplication of effort and repetition of text in the specification, certain features are described in relation to only one or several aspects or embodiments of the invention. However, it is to be understood that, where it is technically possible, features described in relation to any aspect or embodiment of the invention may also be used with any other aspect or embodiment of the invention.

### Brief Description of the Drawings

For a better understanding of the present invention, and to show more clearly how it may be carried into effect, reference will now be made, by way of example, to the accompanying drawings, in which:
Figure 1 is a schematic side sectional view a windscreen display system according to an arrangement of the present disclosure;
Figure 2 is a schematic top view of the windscreen display system according to the arrangement of the present disclosure;
Figures 3a and 3b are schematic front views showing a first image and a second image respectively projected onto the windscreen; and
Figure 4 is a side sectional view of a filter according to the arrangement of the present disclosure.

### Detailed Description

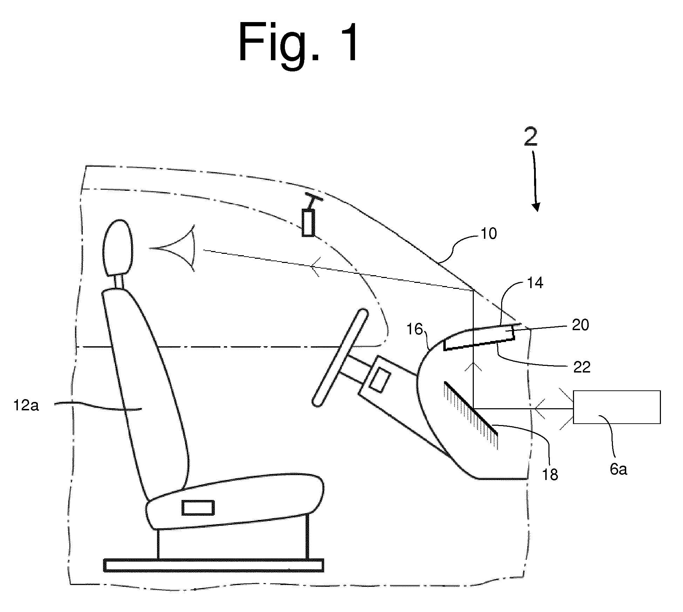

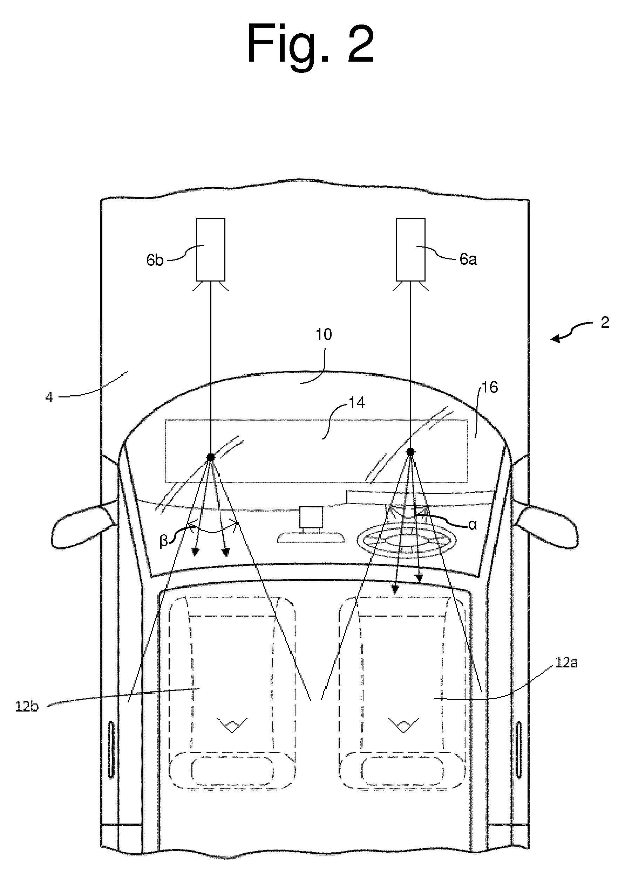

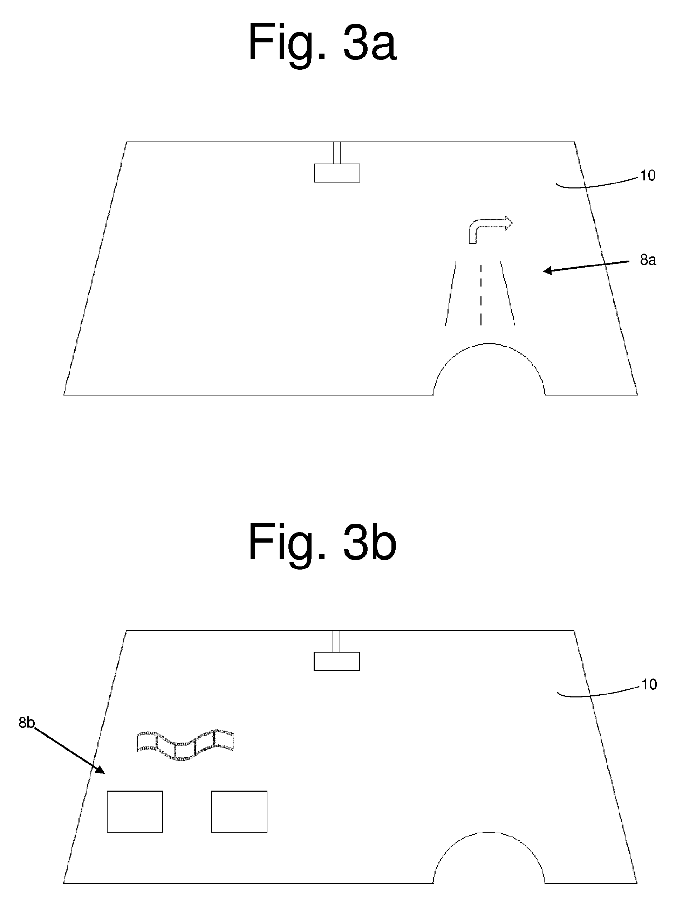

With reference to Figures 1 and 2 a windscreen display system 2 for a motor vehicle 4 comprises a first projector 6a and a second projector 6b. As depicted in Figure 3a, the first projector 6a is configured to project a first image 8a onto a driver's side of a windscreen 10. By contrast, as depicted in Figure 3b, the second projector 6b is configured to project a second image 8b onto a passenger's side of the windscreen 10. Accordingly, light from the first projector 6a is reflected by the windscreen 10 towards the driver and light from the second projector 6b is reflected by the windscreen 10 towards the passenger of the vehicle. The driver of the vehicle may sit in a driver's seat 12a and the passenger may sit in a passenger's seat 12b, which is beside the driver's seat 12a.

The first and second projectors 6a, 6b may be separate components or they may be combined into a single projector that is capable of emitting two images and directing the two images in different directions. The projectors may comprise a Light Emitting Diode (LED), Liquid Crystal Display (LCD) or Digital Light Processing (DLP) projector or any other image projector.

The windscreen display system 2 further comprises one or more privacy filters 14 disposed between the windscreen 10 and the first and second projectors 6a, 6b. In other words, light emitted from the first and second projectors 6a, 6b passes through the privacy filter 14 before reaching the driver or passenger. In a particular arrangement the privacy filters 14 are provided in the light path between the first and second projectors 6a, 6b and the windscreen 10. Furthermore, the privacy filters may be spaced apart from the first and second projectors 6a, 6b and the windscreen 10. The privacy filters 14 are configured to reduce or limit a viewing angle of the light passing through the privacy filters. The privacy filters 14 are configured such that light passing through the privacy filters and reflecting from the windscreen is viewable by only one of the driver and the passenger.

The privacy filters 14 are configured to permit light rays that are orthogonal to the surface of the privacy filter to pass through. Light rays that are otherwise inclined to this orthogonal direction may still pass through the privacy filters 14 if the angle of inclination to the orthogonal direction is less than a threshold angle. Therefore, although the privacy filter may substantially linearise the light passing through the privacy filter, an amount of dispersed light may still pass through. The privacy filter may be configured and/or positioned such that the resulting dispersion of light is within the field of view of one of the driver and passenger, but not the other of the driver and passenger. In particular, the spacing of the privacy filter from the windscreen and/or the spacing of the privacy filter from the projector may be configured such that the resulting dispersion of light is within the field of view of one of the driver and passenger, but not the other of the driver and passenger. Accordingly, the privacy filters 14 may be arranged so as to limit the field of view in a lateral direction of the vehicle. The field of view in other directions may be unaffected by the privacy filter.

The privacy filter 14 may comprise any commercially available privacy filter, such as the privacy filters provided by 3M (RTM). The privacy filter may comprise a pair of polarizing layers orientated with polarisation axes at 90° to one another and a pair of half-waveplates provided between the polarizing layers. Each half waveplate may comprise strips of birefringent material interspersed between isotropic portions. The birefringent portions of one of the half-waveplates may be staggered in relation to the birefringent portions of the other half-waveplate. The birefringent portions of either half-waveplate may rotate light by 90°. Accordingly, light that is substantially orthogonal to the privacy filter passes through first polarizing layer, a birefringent portion of one of the half-waveplates, an isotopic portion of the other half-waveplate and then the second polarizing layer which is orientated at 90° to the first polarizing layer. By contrast, light that is oblique to the orthogonal direction will pass through two isotropic portions or two birefringent portions and as a result will be rotated by 0° or 180° respectively and will not be able to pass through the second polarizing layer. Accordingly, the privacy filters 14 may be similar to that disclosed in US 6,239,853 B1.

As shown in Figure 2, light from the first projector 6a may disperse from the privacy filter 14 with a field of view angle α. The passenger is outside the field of view of the light rays from the first projector that pass through the privacy filter. Light from the second projector 6b may disperse from the privacy filter 14 with a field of view angle β. The driver is outside the field of view of the light rays from the second projector that pass through the privacy filter.

In the arrangement depicted in Figures 1 and 2, the projectors 6a, 6b may be provided within or behind a dashboard 16 of the vehicle. In the particular arrangement shown, the first projector 6a is provided substantially in line with the driver and the second projector 6b is provided substantially in line with the passenger. As shown, the projectors 6a, 6b may emit light in a rearwards direction, however, it is equally envisaged that the projectors may emit light in a lateral or upwards direction or a combination of any of these directions.

Referring to Figure 1, the windscreen display system 2 may further comprise a mirror 18, which is positioned to redirect light from the projectors 6a, 6b onto the windscreen 10. In the particular arrangement shown, the mirror 18 may be angled at 45° to the horizontal, although it will be appreciated that the mirror may be disposed at other angles. Each of the first and second projectors 6a, 6b may be provided with a separate mirror 18, although a single mirror extending across at least a portion of the width of the vehicle may be provided. The mirror may redirect a substantially horizontal light ray from one of the projectors to a substantially vertical direction. The windscreen 10 may subsequently reflect this light ray in a substantially horizontal direction towards the driver or passenger.

In the depicted arrangement, the projectors 6a, 6b are set back from the dashboard front. However, such an arrangement may impinge on an engine or storage bay at the front of the vehicle. Accordingly, it is equally envisaged that the projectors 6a, 6b may project light in a lateral direction that is subsequently redirected by one or more mirrors in an upwards direction towards the windscreen 10. If the projectors 6a, 6b emit light in a lateral direction of the vehicle, the projectors may be provided closer to the front of the dashboard. Alternatively, the projectors could project light in an upwards direction and the mirrors could be omitted.

The privacy filters 14 may be provided on the top of the dashboard 16. There may be a single privacy filter 14 that extends across the width of the vehicle and thus receives light from both the first and second projectors 6a, 6b. However, it is equally envisaged that separate privacy filters 14 may be provided, one for each of the first and second projectors 6a, 6b. The privacy filters 14 may be substantially horizontal or they be inclined relative to the horizontal.

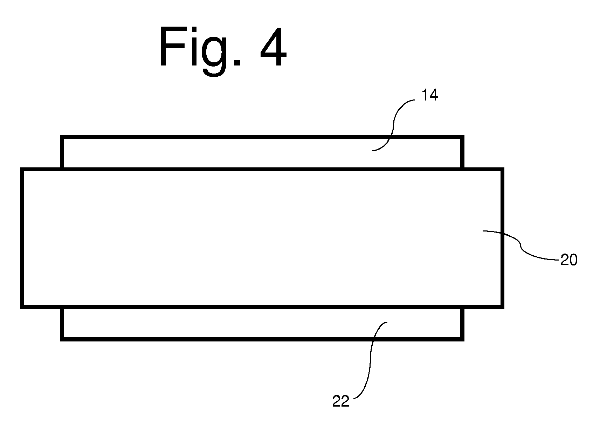

With reference to Figure 4, the privacy filters 14 may be applied to a transparent layer 20, which may be made from glass or a plastic, such as Perspex (RTM). The transparent layer 20 may be flush with the dashboard and the transparent layer may be flat or curved following the contours of the dashboard. The privacy filter 14 may be applied to the top surface of the transparent layer 20.

The windscreen display system 2 may further comprise a rear projection display layer 22. The rear projection display layer 22 may assist in diffusing the otherwise specular light emitted from the projectors 6a, 6b. The rear projection display layer may comprise a commercially available rear display film, such as those provided by 3M (RTM). For example, the rear projection display layer may comprise microscopic transparent spheres that are arranged to diffuse the incident light. As depicted in Figure 4, the rear projection display layer 22 may be provided on an underside of the transparent layer 20. Accordingly, light from the projectors 6a, 6b may be diffused by the rear projection display layer 22 before passing through the transparent layer 20 and the privacy filter 14. The privacy filter 14 serves to reduce the viewing angle of the light diffused from the rear projection display layer 22.

In addition to the privacy filter 14, the windscreen display system 2 may further comprise a tinted layer applied to an inner surface of the windscreen 10. The tinted layer may comprise a tinted coating or a tinted film applied to the windscreen 10. The tinted layer may serve to reduce the amount of light from the projectors 6a, 6b passing through the windscreen 10 and reflecting on an outer surface of the windscreen. Such reflections are undesirable as they can appear as a duplicate image. The tinted layer advantageously reduces such additional reflections and promotes the reflection of the lights from the projectors on the inner surface of the windscreen. In order to comply with legislative requirements, the tinted layer may permit at least 75% of light through the windscreen.

The windscreen 10 may not be flat. Therefore, to counteract any distortion due to curvature of the windscreen, the projector 6a, 6b may project a distorted image that once reflected by the windscreen appears to the driver or passenger to be undistorted. Additionally or alternatively, the mirror 18 may be curved in such a way so as to counter the distortion caused by curvature of the windscreen.

The windscreen display system 2 may further comprise a controller configured to control each of the projectors 6a, 6b. The controller may control the content of the images 8a, 8b. The controller may also control the brightness of the images, e.g. to account for external light levels.

As depicted in Figure 3a the driver may see the first image 8a and not the second image 8b. The first image may comprise navigational information. The navigational information may be overlaid on the road ahead. The first image 8a may thus provide minimal distraction to the driver and may ensure that the driver is focussed on the road or obstacles ahead. By contrast, as depicted in Figure 3b, the passenger may be able to view the second image 8b but not the first image 8a. The passenger may therefore enjoy media based content without being distracted by navigational information. The passenger may also be able to view the road ahead through the driver's side of the windscreen, and this may assist in reducing occurrences of travel sickness. The media based content in the second image 8b may comprise moving images, such as films, data relating to music currently being played, social media, internet base content and/or any other media. For example the second image may comprise messages, e-mails, or images from any software package, such as a word processor. The second image may be linked to a portable device, such as a smart phone, associated with the passenger.

It will be appreciated by those skilled in the art that although the invention has been described by way of example, with reference to one or more examples, it is not limited to the disclosed examples and alternative examples may be constructed without departing from the scope of the invention as defined by the appended claims.

## Claims

1. A windscreen display system (2) for a motor vehicle (4), the windscreen display system comprising:
a first projector (6a) configured to project a first image onto a driver's side of a windscreen (10) so that light from the first projector is reflected by the windscreen towards the driver;
a second projector (6b) configured to project a second image onto a passenger's side of the windscreen (10) so that light from the second projector is reflected by the windscreen towards the passenger; and
one or more privacy filters (14) disposed between the windscreen (10) and the first and second projectors (6a, 6b), the privacy filters configured to limit the viewing angle of the light emitted from the first and second projectors such that, after passing through the privacy filters and reflecting from the windscreen, the first image is not viewable by the passenger and the second image is not viewable by the driver.

2. The windscreen display system (2) of claim 1, wherein the windscreen display system further comprises one or more mirrors (18) configured to redirect light emitted from the first and/or second projectors (6a, 6b) in the direction of the windscreen (10).

3. The windscreen display system (2) of claim 2, wherein one or more of the privacy filters (14) is provided between the one or more mirrors (18) and the windscreen (10).

4. The windscreen display system (2) of claim 2 or 3, wherein the windscreen display system comprises a mirror (18) common to each of the first and second projectors (6a, 6b).

5. The windscreen display system (2) of claim 2 or 3, wherein the windscreen display system comprises one mirror (18) for each of the first and second projectors (6a, 6b).

6. The windscreen display system (2) of any of claims 2 to 5, wherein one or more of the mirrors (18) are curved so as to correct any distortion resulting from a non-flat windscreen (10) such that after reflection by the windscreen the first or second images appear to the respective driver or passenger to be undistorted.

7. The windscreen display system (2) of any of the preceding claims, wherein the first and/or second projectors (6a, 6b) are configured to project a distorted image that after reflection by the windscreen (10) appears to the respective driver or passenger to be undistorted.

8. The windscreen display system (2) of any of the preceding claims, wherein one or more of the privacy filters (14) are provided on a top of a dashboard (16) of the vehicle.

9. The windscreen display system (2) of any of the preceding claims, wherein the windscreen display system comprises a privacy filter (14) common to each of the first and second projectors (6a, 6b) or one privacy filter for each of the first and second projectors.

10. The windscreen display system (2) of any of the preceding claims, wherein the windscreen display system comprises a windscreen (10) comprising a tinted layer on an inner surface of the windscreen.

11. The windscreen display system (2) of claim 10, wherein the tinted layer comprises a coating or film applied to the windscreen (10).

12. The windscreen display system (2) of any of the preceding claims, wherein the windscreen display system comprises one or more rear projection display layers (22) disposed between the projectors (6a, 6b) and the privacy filters (14).

13. The windscreen display system (2) of claim 12, wherein the windscreen display system further comprises one or more transparent layers (20) to which the rear projection display layers (22) and privacy filters (14) are attached.

14. A vehicle (4) comprising the windscreen display system (2) of any of the preceding claims.

15. A method of displaying images on a windscreen (10) of a motor vehicle (4), the method comprising:
projecting a first image onto a driver's side of the windscreen (10) with a first projector (6a) so that light from the first projector is reflected by the windscreen towards the driver;
projecting a second image onto a passenger's side of the windscreen (10) with a second projector (6b) so that light from the second projector is reflected by the windscreen towards the passenger; and
limiting the viewing angle of the light emitted from the first and second projectors (6a, 6b) with one or more privacy filters (14) disposed between the windscreen (10) and the first and second projectors, the privacy filters being configured to limit the viewing angle of the light emitted from the first and second projectors such that, after passing through the privacy filters and reflecting from the windscreen, the first image is not viewable by the passenger and the second image is not viewable by the driver.
